(19) Europäisches Patentamt

European Patent Office

Office européen des brevets

(11) **EP 1 495 136 B1**

(12) **EUROPEAN PATENT SPECIFICATION**

(45) Date of publication and mention
of the grant of the patent:
**06.12.2006 Bulletin 2006/49**

(51) Int Cl.:
**C12Q 1/68** (2006.01)

(21) Application number: **03704733.9**

(22) Date of filing: **10.01.2003**

(86) International application number:
**PCT/GB2003/000079**

(87) International publication number:
**WO 2003/070980 (28.08.2003 Gazette 2003/35)**

(54) **NUCLEIC ACID QUANTIFICATION**

NUKLEINSÄUREQUANTIFIZIERUNG

QUANTIFICATION D'ACIDES NUCLEIQUES

(84) Designated Contracting States:
**AT BE BG CH CY CZ DE DK EE ES FI FR GB GR
HU IE IT LI LU MC NL PT SE SI SK TR**

(30) Priority: **20.02.2002 GB 0204022**

(43) Date of publication of application:
**12.01.2005 Bulletin 2005/02**

(73) Proprietor: **Onyvax Limited**
**London SW17 0RE (GB)**

(72) Inventors:
• **WHELAN, Michael**
**Steventon,**
**Oxon OX13 6SP (GB)**
• **WHELAN, Joseph**
**London W6 9HF (GB)**

(74) Representative: **Roberts, Michael Austin**
**Reddie & Grose,**
**16 Theobalds Road**
**London WC1X 8PL (GB)**

(56) References cited:
**EP-A- 1 138 783**

• **HEIN J ET AL: "QUANTIFICATION OF MURINE
IFN-GAMMA MRNA AND PROTEIN EXPRESSION:
IMPACT OF REAL-TIME KINETIC RT-PCR USING
SYBR GREEN I DYE" SCANDINAVIAN JOURNAL
OF IMMUNOLOGY, BLACKWELL SCIENCE
PUBL., OXFORD, GB, vol. 54, no. 3, 2001, pages
285-291, XP009014576 ISSN: 0300-9475 cited in
the application**
• **A. NITSCHE, N. STEUER, C.A. SCHMIDT, O.
LANDT, W. SIEGERT: "Different Real-time PCR
formats compared for the quantitative detection
of humen cytomegalovirus DNA" CLINICAL
CHEMISTRY, vol. 45, no. 11, 1999, pages
1932-1937, XP002254208**
• **T.D. SCHMITTGEN, B.A. ZAKRAJSEK: "Effect of
experimental treatment on housekeeping gene
expression: validation by real-time, quantitative
RT-PCR" JOURNAL OF BIOCHEM. BIOPHYSIS.
METHODS, vol. 46, 2000, pages 69-81,
XP002254209 cited in the application**
• **OVERBERGH L ET AL: "Quantification of murine
cytokine mRNAs using real time quantitative
Reverse Transcriptase PCR" CYTOKINE,
ACADEMIC PRESS LTD, PHILADELPHIA, PA, US,
vol. 11, no. 4, April 1999 (1999-04), pages 305-312,
XP002227388 ISSN: 1043-4666**
• **V.L. SINGER, L.J. JONES, S. T. YUE, R. P.
HAUGLAND: "Characterization of PicoGreen
reagent and development of a fluorescence-
based solution assay for double-tranded DNA
quantitation" ANALYTICAL BIOCHEMISTRY, vol.
249, 1997, pages 228-238, XP002254210**

SEQUENCE LISTING

<110> Onyvax Limited et al.

<120> Nucleic Acid Quantification

<130> 44163/JMD/MR

<140> PCT/GB2003/000079
<141> 2003-01-10

<150> GB0204022.8
<151> 2002-02-20

<160> 14

<170> PatentIn version 3.1

<210> 1
<211> 22
<212> DNA
<213> Artificial Sequence

<220>
<223> Synthetic oligonucleotide

<400> 1
atgtattgct ttgcgttgga ca          22

<210> 2
<211> 24
<212> DNA
<213> Artificial Sequence

<220>
<223> Synthetic oligonucleotide

<400> 2
tcaatagcaa caaaaagaaa cgag          24

<210> 3
<211> 34
<212> DNA
<213> Artificial Sequence

<220>
<223> Synthetic oligonucleotide
<220>
<221> misc_feature
<222> (1)..(1)
<223> 6Fam

<220>
<221> misc_feature
<222> (34) .. (34)
<223> Tamra

<400> 3
tcaagtcagt taccgaataa ttagtcagct tttc          34

<210> 4
<211> 24
<212> DNA
<213> Artificial Sequence

<220>

<223> Synthetic oligonucleotide

<400> 4
tcaccaggat gctcacattt aagt          24

<210> 5
<211> 29
<212> DNA
<213> Artificial Sequence

<220>
<223> Synthetic oligonucleotide

<400> 5
gaggtttgag ttcttcttct agacactga          29

<210> 6
<211> 26
<212> DNA
<213> Artificial Sequence

<220>
<223> Synthetic oligonucleotide
<220>
<221> misc_feature
<222> (1)..(1)
<223> 6Fam

<220>
<221> misc_feature
<222> (26)..(26)
<223> Tamra

<400> 6
atgcccaaga aggccacaga actgaa          26

<210> 7
<211> 24
<212> DNA
<213> Artificial Sequence

<220>
<223> Synthetic oligonucleotide

<400> 7
caaggactcc tttaacaaca agtt          24

<210> 8
<211> 20
<212> DNA
<213> Artificial Sequence

<220>
<223> Synthetic oligonucleotide

<400> 8
gagatgcctt cagcagagtg          20

<210> 9
<211> 17
<212> DNA
<213> Artificial Sequence

<220>
<223> Synthetic oligonucleotide
<220>
<221> misc_feature
<222> (1)..(1)
<223> 6Fam
<220>
<221> misc_feature
<222> (17)..(17)
<223> MGB

<400> 9
cagctgatcc ttcattt          17

<210> 10
<211> 19
<212> DNA
<213> Artificial Sequence
<220>
<223> Synthetic oligonucleotide

<400> 10
ctgccccaat ccctttatt          19

<210> 11
<211> 20
<212> DNA
<213> Artificial Sequence
<220>
<223> Synthetic oligonucleotide

<400> 11
cccaattctc tttttgagcc          20

<210> 12
<211> 28
<212> DNA
<213> Artificial Sequence
<220>
<223> Synthetic oligonucleotide
<220>
<221> misc_feature
<222> (1)..(1)
<223> 6Fam

<220>
<221> misc_feature
<222> (28)..(28)
<223> Tamra

<400> 12
cccctccttc agacaccctc aacctctt          28

<210> 13
<211> 19
<212> DNA
<213> Artificial Sequence

<220>
<223> Synthetic oligonucleotide

<400> 13
tcgacagtca gccgcatct        19

<210> 14
<211> 19
<212> DNA
<213> Artificial Sequence

<220>
<223> Synthetic oligonucleotide

<400> 14
ccgttgactc cgaccttca        19

**Description**

The present invention relates to quantification of nucleic acids using real-time PCR.

Conventional reverse-transcription polymerase chain reaction (RT-PCR) is a sensitive and widely used technique. It has often been applied to the detection of relatively rare mRNA transcripts. However, it is not truly quantitative and is often a gross underestimate of total message levels actually present, mostly due to depletion of reagents during the reaction (Santagati *et al,* 1997). Consequently, when PCR products are visualised on a gel, the intensity of the bands are not normally proportional to the amount of initial target, thus making image analysis inaccurate.

One of the most accurate methods to overcome this limitation is to use the 5'-3' exonuclease reaction as exemplified by the ABI Prism 7700 sequence detector (Holland *et al.* 1991; Livak *et al,* 1995). In "real-time PCR" a third element is present, in addition to the standard PCR primers, called the probe. This probe lies downstream of the forward primer and has a fluorescent tag at the 5' end and a quencher at the 3' end. As the PCR proceeds, Taq polymerase uses its 5'-3' exonuclease activity and destroys the probe thus releasing colour. The colour is directly proportional to the amount of target DNA present. Hence, if colour is rapidly detected then large amounts of DNA are present and vice versa when colour release is slow.

Despite the exquisite sensitivity of the real-time PCR reaction, there still exists a major area of contention. In order to compare samples, it is necessary to use some form of calibration. One common method is to employ a reference or "house keeping" gene; the logic being that this will not change even when the target gene alters in level dramatically (Overbergh *et al,* 1999). This is essentially an external control. Whilst this is common practice, there are still significant drawbacks when using this system, most commonly associated with the fact that the reference gene is not truly static and may alter slightly during cellular turnover. Another potential source of discrepancy arises during cDNA synthesis. Ideally, this should be done in a single batch to avoid any potential variability caused by differences in the efficiency of target and reference gene transcription. This is clearly impractical in a clinical setting with samples arriving at different time intervals, for example.

An alternative method of calibration is to employ an internal reference standard in the form of a plasmid containing the target gene. Thus, a known amount of plasmid may be used to construct a calibration curve and then unknown samples identified from this (Li and Wang, 2000). Hence, results may be expressed as a number of copies of a particular gene since copy number is essentially molarity for plasmids. However, to date, this method has not' been ideal, particularly since it relies on construction of reliable standards in conjunction with highly accurate ds-DNA quantitation. One reagent capable of highty accurate dsDNA quantitation is Pica Green®, which is disclosed in Singer et al. (1997).

The present invention provides a new method for quantification of a target nucleic acid molecule which addresses these prior art problems.

According to the present invention, there is provided a method for quantification of a target nucleic acid in a sample, comprising the steps of:

(1) accurately quantifying the total amount of nucleic acid in the sample using PicoGreen® dsDNA quantitation reagent in combination with a known reference standard;
(2) amplifying the target nucleic acid using real-time PCR primers and probes to produce an amount of amplicon;
(3) building a reference plasmid based on this amplicon;
(4) constructing a fluorescance reference curve of real-time PCR reactions with different known quantities of the plasmid using a fluorescent probe;
(5) running a real-time PCR reaction with an aliquot of the sample to amplify the target nucleic acid and obtain a fluorescence result from the binding of the fluorescent probe; and
(6) correlating the fluorescence result of step (5) with the reference curve of step (4) to quantify the target nucleic acid by copy number per amount of total nucleic acid present in the sample (for example, copy number per $\mu$g cDNA).

The use of a target nucleic acid amplicon (an amplified fragment obtained using real-time PCR primers) for construction of a fluorescence reference curve provides an internal control for accurate quantification of target nucleic acid in a sample and does not require the use of a non-target calibration gene. The vast majority of real-time PCR data in the literature is derived from the use of relative quantitation by employing calibration genes. This is fraught with complexity and it is possible to advance arguments against the use of most of these, as discussed further in the Experimental section below. The present invention avoids such problems.

A further advantage of using an amplicon as internal control is that amplification of the target nucleic acid and the control is more likely to be at the same efficiency and the quantification results more accurate.

Step (2) may be conducted as a real-time PCR reaction or as a normal PCR reaction (i.e. without probe).

In the invention, the amplicon of step (2) is cloned into a vector (i.e. a plasmid) to produce a cloned amplicon which is quantified and used to construct the fluorescence reference curve of step (4). Although it is known to construct a reference curve with a plasmid into which the target gene has been inserted (see for example Li and Wang, 2000), it is usual to clone the entire target gene, or at least a PCR fragment much larger than the amplicon produced by real-time PCR (an amplicon is typically 50-150 bp in length). We have shown that this is unnecessary, since the PCR primers used for real-time PCR can also be used for cloning. Thus, products from a real-time run can simply be cloned into a vector and a reference plasmid constructed. The simplicity of this technique makes the use of reference genes, and their associated complexity, redundant. In a preferred embodiment, the real-time PCR reaction in step (5) may employ the real-time primers used in step (2). This will mean that the method requires fewer reagents and also that control and target amplification will be essentially identical.

The nucleic acid quantification method of step (1) may have a resolution of at least of 1 ng/ml DNA. The nucleic acid quantification method employs PicoGreen (RTM) dsDNA quantitation reagent. The use of a sensitive nucleic acid quantification method is important for accurate quantification of target nucleic acid. The nucleic acid quantification method is fluorescent-based (as with the PicoGreen reagent). The method may be performed such that both step (1) and step (5) are carried out using a fluorescence-detecting thermocycler, for example a Perkin Elmer/Applied Biosystems Prism 7700 Sequence Detector System. Alternatively, step (1) could be performed using a fluorimeter. The use of the same machine for step (1) and step (5) simplifies the method and provides for faster analysis. For example, if both DNA quantification and PCR are carried out using the ABI Prism 7700, the need for extra equipment is reduced. A high throughput may be maintained using a 96-well format, for example with the ABI Prism 7700.

In a preferred embodiment, the fluorescence reference curve may be constructed using the natural log of the threshold cycle (cT) of the real-time PCR reactions against given quantities of the amplicon or cloned amplicon expressed as copy number. The target nucleic acid in the sample is quantified as copy number per amount of total nucleic acid present in the sample (for example, copy number per $\mu$g cDNA). The advantage of this is that it renders any losses that can occur for example during mRNA isolation from different samples irrelevant and thus eliminates the need for relative quantitation using house-keeping gene analysis. Furthermore, with results expressed as copy number/ quantity (e.g. $\mu$g) of cDNA, comparison between different experiments and samples will be possible. This will be particularly advantageous when comparing results from experiments detecting amplicons of different length.

The target nucleic acid may comprise RNA, for example mRNA. In this case cDNA may be constructed from the RNA and the cDNA used as the target nucleic acid in steps (2) and (5). This system will allow quantification of gene expression levels in cells.

In one embodiment, protein levels in the sample are quantified. For example, the supernatant from cell samples used to generate cDNA may be retained and the protein level quantified. In this way, both mRNA and protein levels may be obtained from the same sample. This is ideally suited to clinical trial analysis where multiple samples must be compared over time with great accuracy.

The target nucleic acid used in step (2) may be from a source other than the sample.

For accurate calculations, steps (4) and (5) are conducted in same (real-time PCR) reaction plate.

The method has been demonstrated (see below) to be particularly useful to measure cytokine levels. Thus the invention also provides a method for use when the quantity of target nucleic acid is directly proportional to a cytokine quantity. The real-time PCR reactions may employ pre-defined assay reagents (PDAR) specific for IFN-$\gamma$ or IL4 (for example, as obtainable from Applied Biosystems). Alternatively, the real-time PCR reactions may use primers and probes for IEN-$\gamma$, IL2, IL10 and/or TNF-$\alpha$ as defined in Table 1 below (SEQ ID NOs: 1-12).

The method uses a real-time PCR probe. However, alternative systems such as a dye-alone system (for example dsDNA specific dyes such as SYBR-Green) are not excluded. Furthermore, it may be possible to use nucleic acid quantification methods in which absorbance rather than fluorescence is detected, allowing for construction of absorbance reference curves rather than fluorescence reference curves.

Specific embodiments of the invention will be further described below with reference to the accompanying figures, of which:

Fig. 1 Shows an example of a standard curve obtained for total DNA quantitation. Linear equation: y = 0.0249x - 0.5022, $R^2$ = 0.9983;

Fig. 2 Shows an example of a standard curve obtained for specific DNA quantitation. The linear equation is: y=-0.0723x-3.8854, $R^2$ = 0.9974;

Fig. 3 Shows total cDNA quantitation of mouse splenocytes stimulated for 48 hours;

Fig. 4 Shows IEN-$\gamma$ mRNA quantification in mouse splenocytes cultured for 48 hours (average of 2 runs);

Fig. 5 Shows IL-4 mRNA quantification in mouse splenocytes cultured for 48 hours (average of 2 runs);

Fig. 6 Shows a further example of standard curve obtained for total DNA quantification. Linear equation: y = 0.0327x

-1.8695, $R^2$ = 0.9988;

Fig. 7 Shows total cDNA quantification of human PBL stimulated for 24 hours (average of 2 runs);

Fig. 8 Shows the ratio of average $C_T$ value compared to medium control cDNA in ribosomal and GAPDH real time PCRs. ⊡ = medium; ⊡ = ConA; □= PMA + Ionomycin; ⦀ = LPS;

Fig. 9 Shows cytokine levels measured using Cytometric Bead Array (**A**), absolute cDNA quantification (**B**) and quantification relative to ribosomal cDNA (**C**). ▨ = IFN-γ, ⊡ = TNF-α, □ = IL10; ⊠ = IL2;

Fig. 10 Shows IFN-γ copy number quantification of serially diluted cDNA; and

Fig. 11 Shows RT-PCR of serially diluted IFN-γ plasmid standard (circular (**A**) and linearised (**B**)) diluted in the range of 1 in $10^4$ to 1 in 5 x $10^9$. For **A,** linear equation: y = -0.0612x + 3.7789, $R^2$ = 0.9956. For **B,** linear equation: y = -0.062x + 3.6938, $R^2$ = 0.9919.

## Experimental

We describe a preferred method to quantitate the copy number of cytokine molecules within cDNA samples by including a protocol capable of accurately measuring the total amount of DNA present in samples with high precision. The method allows the expression of results as copy number per μg of cDNA. The technique overcomes many of the objections often advanced against real-time data and we believe is simple, and routine enough, for standard use in clinical laboratories. Although we have examined cytokine levels in particular, this technique is applicable to more general real-time PCR applications.

**Materials and Methods**

Splenocyte extraction and culture (Example 1)

Spleens were dissected from C57/b16 mice (St. George's Hospital Medical School, London, UK). Splenocytes were isolated by passing through a 40μm nylon cell strainer (Falcon, Oxford, UK) and washing with medium (RPMI 1640 medium (Sigma Chemical Co., Dorset, UK) supplemented with 10% foetal calf serum (FCS; PAA Laboratories, Teddington, UK), L-glutamine (Life Technologies, Paisley, Scotland), penicillin (1000 U/ml) and streptomycin (1000μg/ml) (Life Technologies)).
Peripheral blood lymphocytes (pbl) were removed by centrifugation over Histopaque 1083 (Sigma). Cells from the interface were washed with phosphate buffered saline (PBS) before culturing at a concentration of 2.5 x $10^6$/ml in the presence of Concanavalin A (ConA) (2.5μg/ml; Sigma), ConA plus recombinant interferon-γ (0.01μg/ml, R&D Systems, Abingdon, UK) or ConA plus recombinant interleukin-4 (0.02μg/ml, R&D systems). A control culture was set up simultaneously containing medium and cells alone. Cultures were incubated for 48 hours in a 37°C 5% $CO_2$ atmosphere incubator before washing in PBS and proceeding with extraction of RNA.

Human peripheral blood lymphocyte extraction and culture (Example 2)

10ml human blood from a healthy donor was diluted with 10ml phosphate buffered saline (PBS) and peripheral blood lymphocytes (PBL) were removed by centrifugation over Histopaque 1077 (Signa). Cells from the interface were washed with PBS before culturing at a concentration of 2.5 x $10^6$/ml in the presence of either (A) Concanavalin A (ConA) (2.5μg/ml; Sigma), (B) phorbol 12-myristate 13-acetate (PMA) (0.05μg/ml; Sigma) + Ionomycin (0.5mg/ml; Sigma), (C) lipopolysaccharide (LPS) (1μg/ml; Sigma) or (D) control culture of medium and cells alone. Cultures were incubated in a 37°C 5% $CO_2$ atmosphere incubator. After 24 hours incubation the cultures were transferred to 1.5ml eppendorf tubes and cells pelleted at 8000rpm for 1 minute. Supernatants were transferred to a fresh eppendorf and stored at -80°C prior to analysis by Cytometric Bead Array (CBA). Cell pellets were the source for RNA extraction and cDNA synthesis.

RNA extraction and cDNA synthesis

RNA was extracted from cell pellets of 1x$10^7$ cells (mouse spleen PBL; Example 1) or 2.5x$10^6$ cells (human blood PBL; Example 2) using an Absolutely RNA RT PCR miniprep kit (Stratagene, Amsterdam, Holland) according to manufacturers guidelines. Contaminating genomic DNA was eliminated by the presence of a DNase treatment step in the kit. RNA was eluted in a volume of 31-32$\mu$l and immediately used as template in cDNA synthesis. Reverse transcription was performed in 0.2ml PCR tubes in a total volume of 50$\mu$l containing 10mM dNTPs (Gibco BRL), 1$\mu$g Oligo(dT)(12-18) primer (Invitrogen, Groningen, Holland), 80 units recombinant ribonuclease inhibitor (Invitrogen), 31$\mu$l RNA, 10$\mu$l first strand buffer (5x) (Amersham Pharmacia, Little Chalfont, UK) and 500 units MMLV reverse transcriptase (Amersham Pharmacia). This was incubated at 37°C for 1 hour and cDNA was subsequently stored at 4°C (Example 1) or -20°C (Example 2).

PicoGreen quantification of total DNA

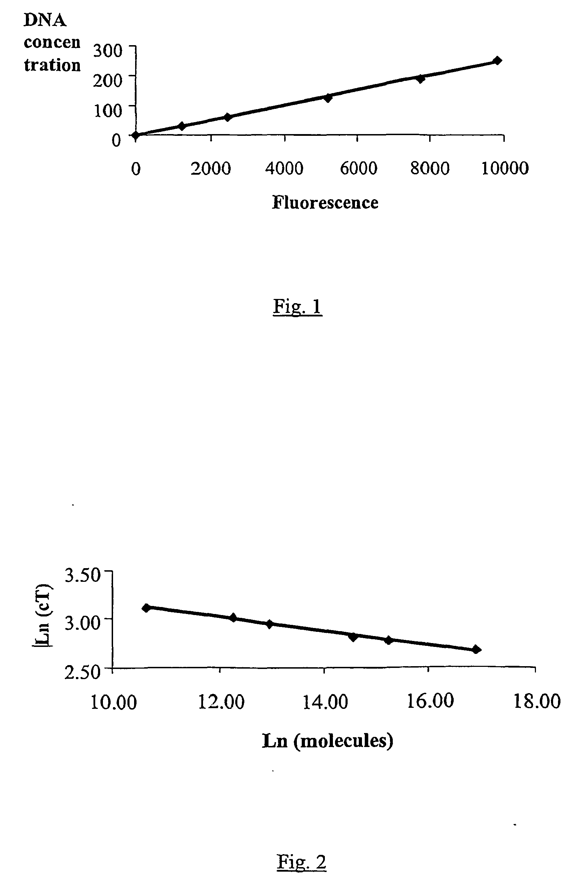

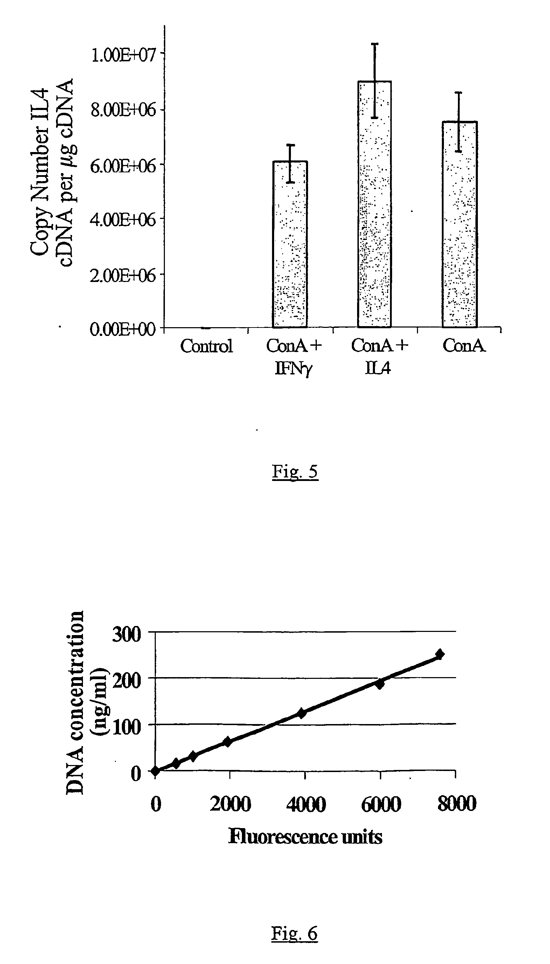

Quantification was performed using reagents from the PicoGreen dsDNA quantitation kit (Molecular Probes, Cambridge, UK). $\lambda$ DNA standard (Molecular Probes) was diluted in 1 x Tris-EDTA (TE) to 500ng/ml and aliquoted in triplicate into 96 well optical reaction plates (Applied Biosystems, Warrington, UK). TE was added to a final volume of 50$\mu$l and final concentrations of 500ng/ml, 375ng/ml, 250ng/ml, 125ng/ml, 62.5ng/ml, 31.25ng/ml and 0ng/ml constructed. cDNA was diluted in 1 x TE and added in triplicate to the reaction plate at 1 in 100 and 1 in 200 dilutions. PicoGreen double stranded DNA quantitation reagent (Molecular Probes) was diluted 1 in 200 and 50$\mu$l was added to each well thus doubling the dilutions of DNA. An optical adhesive cover was fixed over the wells and the plate was then wrapped in aluminium foil to prevent light degradation of the PicoGreen reagent. The plate was vortexed for 3 bursts of 1 or 2 seconds before flicking the plate downwards to force the liquid to the bottom of the wells. The plate was read using a post-PCR read protocol on the ABI Prism 7700 sequence detector under PicoGreen settings. These settings were generated by creating an absorbance spectrum for a PicoGreen standard as per manufacturer's instructions, with excitation at 480nm and measurement at 520nm. A linear standard curve was drawn, plotting the average fluorescence values obtained for the $\lambda$DNA triplicate values against the standard DNA concentration (Example 1 - Fig. 1; Example 2 - Fig. 6). The concentration of test DNA was calculated from the linear equation after adjusting for dilution.

Absolute quantification of specific cDNA

Specific controls were constructed for Interferon-$\gamma$ (IFN-$\gamma$), Interleukin-2 (IL2), Interleukin-4 (IL4) and Interleukin-10 (IL10) by cloning products of real-time PCR reactions obtained from PCR's primed with pre-defined assay reagents (PDAR's) specific for IFN-$\gamma$, IL2, IL4 and IL10 (Applied Biosystems). PDAR's, are reagents containing primers and probes (sequence unavailable from Applied Biosystems) for specific cDNA targets optimised for use with the ABI Prism 7700 and usually designed to span intron-exon boundaries. The products were cloned into a pBAD-TOPO vector using the pBAD TOPO TA cloning kit (Invitrogen). 25ml LB-broth cultures containing single colonies were grown up overnight, shaking at 37°C and plasmid was subsequently purified using the QIAfilter plasmid midiprep kit (Quiagen, Crawley, UK). Plasmid DNA was solubilised in 150$\mu$l TE buffer. DNA sequencing (LARK technologies) confirmed the sequences of the cloned products. cDNA for tumour necrosis factor alpha (TNF-$\alpha$) cloned into the pBR322 vector was available from American Type Culture Collection (ATCC number 39894). Oligonucleotides specific for the cloned products were subsequently designed with primer express software (Applied Biosystems) and used in subsequent RT-PCR reactions (see Table 1).

**Table 1**: Oligonucleotide primer sets for real-time PCR (Example 2)

| cDNA Target | Oligonucleotide (5' to 3') |
|---|---|
| IFN-$\gamma$ | For: ATG TAT TGC TTT GCG TTG GAC A [SEQ ID NO: 1] |
| | Rev: TCA ATA GCA ACA AAA AGA AAC GAG [SEQ ID NO: 2] |
| | Pro: 6Fam-TCA AGT CAG TTA CCG AAT AAT TAG TCA GCT TTT C-Tamra [SEQ ID NO: 3] |
| IL2 | For: TCA CCA GGA TGC TCA CAT TTA AGT [SEQ ID NO: 4] |
| | Rev: GAG GTT TGA GTT CTT CTT CTA GAC ACT GA [SEQ ID NO: 5] |
| | Pro: 6Fam-ATG CCC AAG AAG GCC ACA GAA CTG AA-Tamra [SEQ ID NO: 6] |
| IL10 | For: CAA GGA CTC CTT TAA CAA CAA GTT [SEQ ID NO: 7] |
| | Rev: GAG ATG CCT TCA GCA GAG TG [SEQ ID NO: 8] |
| | Pro: 6Fam-CAG CTG ATC CTT CAT TT-MGB [SEQ ID NO: 9] |
| TNF-$\alpha$ | For: CTG CCC CAA TCC CTT TAT T [SEQ ID NO: 10] |

(continued)

| cDNA Target | Oligonucleotide (5' to 3') |
|---|---|
| | Rev: CCC AAT TCT CTT TTT GAG CC [SEQ ID NO: 11] |
| | Pro: 6Fam-CCC CTC CTT CAG ACA CCC TCA ACC TCT T-Tamra [SEQ ID NO: 12] |
| GADPH | For: TCG ACA GTC AGC CGC ATC T [SEQ ID NO: 13] |
| | Rev: CCG TTG ACT CCG ACC TTC A [SEQ ID NO: 14] |

For, Rev and Pro indicate forward, reverse and probes, respectively. All primers and Fam-Tamra probes were purchased from MWG-Biotech. The IL10 Fam-MGB probe was purchased from Applied Biosystems.

Purified plasmid clones were quantitated using the PicoGreen quantification method (see above) with the minor modification that plasmid DNA was measured at 1 in 1000 and 1 in 2000 dilutions.

With the molecular weight of the plasmid and insert known, it is possible to calculate the copy number as follows:

$$\text{Weight in daltons (g/mole)} = (\text{bp size of ds product})(330 \text{ daltons} \times 2\text{nt/bp})$$

Hence: (g/mole)/Avagadro's number=g/molecule=copy number

(where: bp= base pairs, ds= double stranded, nt= nucleotides).

Knowing the copy number and concentration of plasmid DNA the precise number of molecules added to subsequent real-time PCR runs can be calculated, thus providing a standard for specific cDNA quantification.

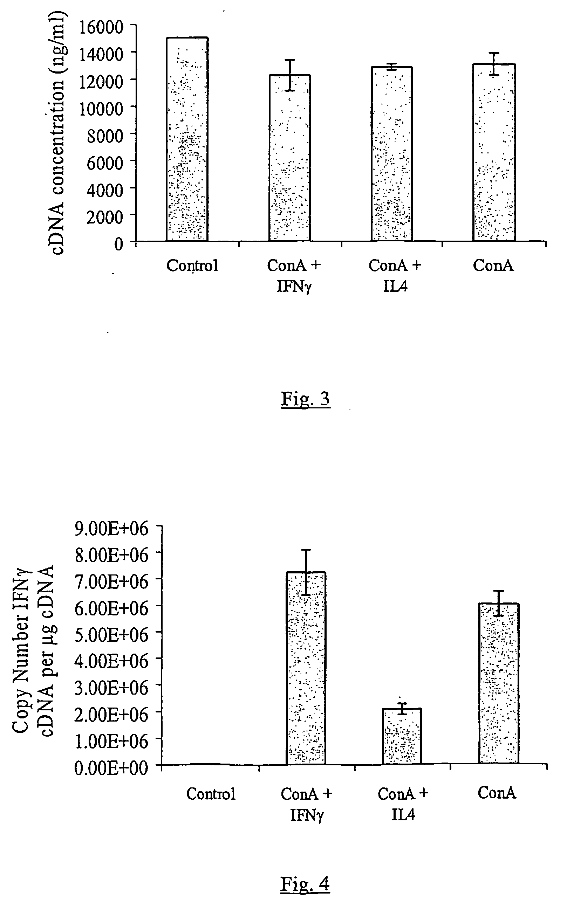

Real-time PCR runs were performed in 96 well optical plates in triplicate wells, each containing 1 x PCR master mix (Applied Biosystems), 1 x PDAR reagent (Applied Biosystems; Example 1) or 0.3pmol/$\mu$l each of forward primer, reverse primer and labeled probe (Example 2), and 2$\mu$l template DNA (either cDNA, cDNA diluted 1 in 5, or plasmid DNA dilutions ranging from 1 in $10^4$ to 1 in $5 \times 10^9$) in a final volume of 25$\mu$l, for 40 cycles using an ABI 7700 Prism (Applied Biosystems). Default 7700 cycle conditions were: 2 minutes at 50°C then 10 minutes at 95°C followed by 40 cycles of 15 seconds at 95°C and 1 minute at 60°C. A standard curve was drawn by plotting the natural log of the threshold cycle ($C_T$) against the natural log of molecules (Example 1; Fig. 2). The $C_T$ was defined as the cycle at which a statistically significant increase in the magnitude of the signal generated by the PCR reaction was first detected. $C_T$ was calculated under default settings for the real time sequence detection software (Applied Biosystems). The equation drawn from the graph was used to calculate the precise number of specific cytokine cDNA molecules present per $\mu$g total cDNA, tested in the same reaction plate as the standard.

Relative quantification

RT-PCR was performed simultaneously for IFN-$\gamma$, IL2, IL10, TNF-$\alpha$, ribosomal cDNA and glyceraldehyde phosphate dehydrogenase (GAPDH) on cDNA diluted 1 in 5 (for cytokine and GAPDH) and 1 in 100 for ribosomal. RT-PCR was performed in 96 well optical reaction plates in triplicate (each reaction containing 1 x SYBR Green PCR Master mix (Applied Biosystems), 0.3pmol/$\mu$l of forward primer and reverse primer (or 1 x ready mixed ribosomal primers (Applied Biosystems) for ribosomal RT-PCR) and 2$\mu$l template cDNA in a final volume of 25$\mu$l). Cycle conditions are the same as described above for absolute quantification of cDNA. GAPDH primers were designed using Primer Express (Applied Biosystems) software (Table 1). Primers for ribosomal cDNA were obtained ready mixed from Applied Biosystems. Fluorescence values were used to calculate the expression of cytokine RNA levels relative to ribosomal and GAPDH RNA.

Linearisation of IFN-$\gamma$ circular plasmid DNA

To determine if circular DNA amplified with a different efficiency to linearised DNA, we cut the IFN-$\gamma$ plasmid standard prior to serial dilution and amplification in parallel to serially diluted circular IFN-$\gamma$ plasmid standard. 0.1$\mu$g plasmid DNA was cut with 10 Units of Eco RV (Life Technologies) in the presence of 'REACT 2' buffer (Life Technologies) for 1 hour at 37°C. The pBAD TOPO vector with IFN-$\gamma$ insert has one Eco RV restriction site at position 3513.

Cytometric Bead Array analysis

Levels of cytokine protein released into the culture medium were measured using the $T_H1/T_H2$ Cytometric Bead Array (CBA) Kit (BD Biosciences, Oxford UK) according to manufacturers instructions. Supernatants of frozen post PBL culture were thawed rapidly and spun down at 2000 x g for 3 minutes to remove any cellular debris. Supernatants were used neat or diluted 1 in 10 in assay diluent (a component of the CBA kit). A mix of the anti-cytokine beads were added to the neat and diluted supernatant samples and incubated with the included PE-detection reagent. Beads and detection reagent were also mixed with a serially diluted standard of all 4 cytokines. Samples and standards were incubated in the dark at room temperature for 3 hours. The samples were analysed on a FACSCalibur fitted with and MAS plate loader (BD Biosciences), and data analysed using CBA analysis software (BD Biosciences).

**Results**

**Example 1: Mouse-derived PBL**

In order to exemplify a preferred method, a first example experiment was carried out as proof of principle. Splenocytes were isolated from a mouse (C57/b16) and then stimulated overnight with ConA alone, ConA and interferon-$\gamma$ or ConA and IL4. The addition of exogenous cytokines causes the *de novo* synthesis of these molecules by the stimulated cells in an autocrine manner (Di Marzio *et al,* 1994; Koch *et al,* 1992). Consequently, these samples may be treated as an example of $T_H1$ and $T_H2$ T-cells and thus are a useful demonstration of the possible uses of this technique.

PicoGreen quantification of total DNA

RNA extracts from cell cultures were reverse transcribed and cDNA quantitated (i.e. quantified) using the PicoGreen quantification method described above. Table 2 illustrates the fluorescence data obtained for the cDNA samples, and the final concentrations were calculated using the equation y = 0.0249x - 0.5022 derived from Fig. 1.

**Table 2**: Calculation of DNA concentration from absorbance values obtained using the PicoGreen quantification method

| Culture | Abs values | Mean Abs | Abs - Zero DNA | Diln cor. | Eqn calc. (ng/ml) | Ave. (ng/ml) |
|---|---|---|---|---|---|---|
| M, 1 in 300 | 2010,2378,2011 | 2133 | 2085 | 625506 | 15700 | 15021 |
| M, 1 in 600 | 971,916,1115 | 1000 | 952 | 571390 | 14342 | |
| Con A + IFN$\gamma$ (i) | 2373,2237,2332 | 2314 | 2266 | 453224 | 11376 | 11465 |
| Con A+ IFN$\gamma$ (ii) | 1189,1158,1249 | 1199 | 1151 | 460319 | 11554 | |
| Con A + IF4 (i) | 2695,2584,2803 | 2694 | 2646 | 529212 | 13283 | 12677 |
| Con A+ IF4 (ii) | 1301,1242,1208 | 1250 | 1202 | 480895 | 12070 | |
| Con A (i) | 2387,2487,2833 | 2569 | 2521 | 504177 | 12655 | 12538 |
| Con A (ii) | 1308,1255,1293 | 1285 | 1237 | 494900 | 12422 | |

Different dilution factors were used for the control sample as limited sample was available. M = medium; Abs = absorbance; Diln cor. = Dilution corrected; Eqn calc. = Equation calculation; Ave. = Average (of equation calculations); (i) = 1 in 200 dilution; (ii) = 1 in 400 dilution.
This method is highly reproducible and quantitation was carried out on two separate occasions, an average taken and error bars drawn (Fig. 3). A standard curve was created each time total DNA quantification was performed to ensure accurate calculations. The equation created however remains relatively unchanged on each occasion. The sensitivity of this method enables minimal DNA to be used in its quantitation, thus preserving valuable samples.

Absolute quantitation of cDNA specific for interferon-$\gamma$ and interleukin-4

Having quantified the total DNA present in the cloned IFN-$\gamma$ and IL4 plasmids, their copy number was calculated and real-time PCR performed on dilutions of these. cDNA from test samples were analysed on the same plate. Fig. 2 illustrates a standard curve and the equation obtained for the IL4 plasmid on one occasion. Table 3 presents the raw $C_T$ values and subsequent copy number calculations using the total cDNA concentrations obtained from the PicoGreen method. The risk of this procedure detecting genomic DNA is eliminated by using a DNase treatment step.

Table 3: Calculation of IL4 cDNA copy number per μg total cDNA using equation calculated from standard curve (Fig. 2)

| Sample | $C_T$ values | Ave. $C_T$ | Copy No. Calc. | Conc. (μg/ml) | Vol. Added (μl) | Amnt Added (μg) | Copy No./μg (molecules/ μg) |
|---|---|---|---|---|---|---|---|
| Control | 20.98 30.75 30.78 | 30.50 | 643.55 | 15.020 | 2 | 0.03004 | 21423 |
| Con A + IFNγ | 20.92 20.38 20.83 | 20.71 | 136315.76 | 12.254 | 2 | 0.02451 | 5562197 |
| Con A + IL4 | 20.11 20.06 20.13 | 20.10 | 206125.49 | 12.842 | 2 | 0.02568 | 8025391 |
| Con A | 20.37 20.26 20.34 | 20.32 | 176911.93 | 13.114 | 2 | 0.02623 | 6745063 |

Figures 4 and 5 illustrate the reproducibility of this method. Quantification was done on two separate occasions, an average taken, and error bars drawn for both quantitation runs of IL4 and IFN-γ. A standard curve should be created each time total absolute cDNA quantification is performed to ensure accurate calculations. The equation created however, will remain relatively unchanged on each occasion. Being a PCR based method, the sensitivity allows minimal cDNA to be used as template and hence screening for a number of cytokines concurrently is feasible. Indeed, it may even be possible to refine this method to multiplex several targets within the same sample, thus examining $T_H1/T_H2$ ratios in the same tube.

### Example 2: Human-derived PBL

In a second experiment, PBL were isolated from a healthy male volunteer and then stimulated overnight with either ConA, PMA + Ionomycin or LPS to induce enhanced cytokine expression under different conditions.

PicoGreen quantification of total DNA

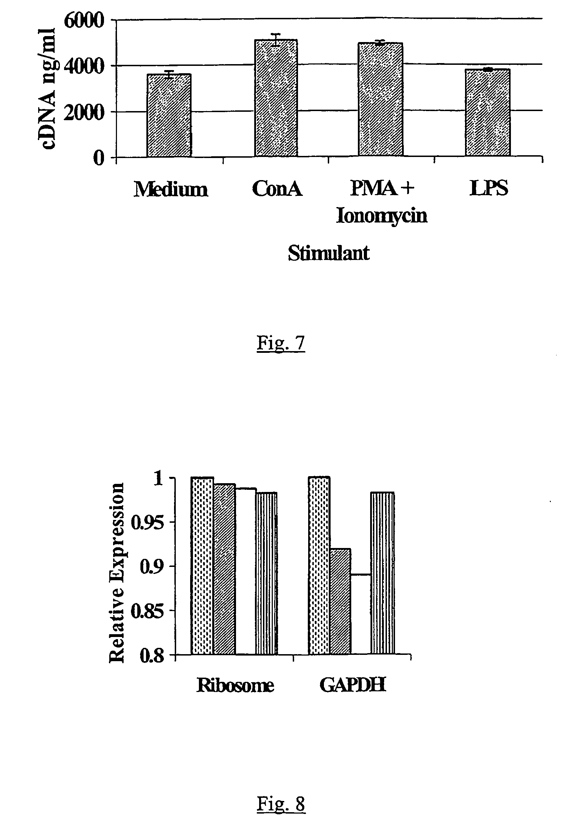

RNA extracts from cell cultures were reverse transcribed and cDNA quantified using the PicoGreen quantification method described above. A standard curve was drawn from which absolute levels of cDNA could be calculated (Fig. 6). In Fig. 6, a λ DNA standard (Molecular Probes) was diluted within the wells of an optical reaction plate to a concentration range of 250ng/ml -15.625ng/ml. Each point plotted is an average of triplicate fluorescence values for every standard concentration measured. The method is highly reproducible and quantification was carried out on two separate occasions for all samples. Average values for cDNA amounts are shown in Fig. 7 (in which two different dilutions of cDNA derived from human PBL stimulated with ConA, PMA + Ionomycin, LPS or medium alone were measured in triplicate on 2 separate occasions and the quantity calculated after adjusting for dilution. The average for each is plotted with standard deviation error bars drawn). A standard curve was created each time total DNA quantification was performed to ensure accurate calculations. The equation created normally remains relatively unchanged on each occasion.

Comparison of ribosomal and GAPDH cDNA

To examine the effect of tissue culture conditions altering the level of house keeping genes, RT-PCR was performed in triplicate on cDNA samples using primers specific for ribosomal or GAPDH cDNA on the same reaction plate. $C_T$ values were used to calculate the relative expression of ribosomal and GAPDH RNA upon stimulation with ConA, PMA + Ionomycin or LPS by dividing the result by the unstimulated (medium) control $C_T$ (see Fig. 8, in which cDNA derived from human PBL stimulated with ConA, PMA + Ionomycin, LPS or medium alone were amplified with primers to ribosomal or GAPDH cDNA. Average $C_T$ values were calculated and divided by those obtained for medium alone samples). Clearly, GAPDH expression was significantly affected by the culture conditions, with only small, but detectable, alterations observed in ribosomal levels.

Comparison of CBA analysis with absolute cDNA quantification and quantification relative to ribosomal RNA levels

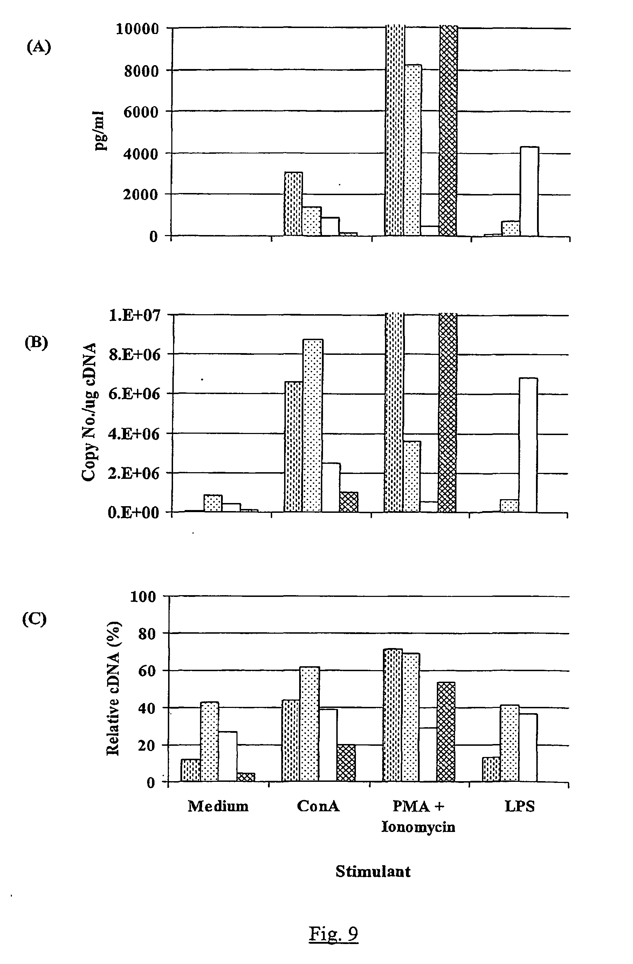

Having quantified the total DNA present in the cloned IFN-$\gamma$, IL2, IL10 and TNF-$\alpha$ plasmids, real-time PCR was performed in triplicate and their copy number was calculated. cDNA from test samples, quantified by the PicoGreen method as described above, was analysed on the same plate in triplicate and their cytokine copy number subsequently calculated (see Fig. 9B. In Fig. 9, human PBL were stimulated with ConA, PMA + Ionomycin, LPS or medium alone for 24 hours. Culture supernatants were used for the CBA analysis. cDNA derived from the PBL were amplified using oligonucleotides specific to IFN-$\gamma$, TNF-$\alpha$, IL10 and IL2 and quantified with the absolute quantification method using cloned cytokine cDNA fragments as standards or quantified relative to ribosomal cDNA). This was compared to the cytokine protein levels from the culture supernatants measured by CBA analysis (Fig. 9A) and levels of cytokine cDNA relative to ribosomal cDNA (Fig. 9C). The scale on the Y-axis for the CBA and absolute quantification graphs was lowered because values for the IFN-$\gamma$ and IL2 cytokines in the PMA + Ionomycin stimulation samples were exceptionally high therefore differences in expression of cytokines in the other samples could not be distinguished. The calculated expression levels for the CBA and absolute cDNA are indicated in Table 4. Interestingly, relative quantification proved fairly robust when a single cytokine was examined, but was poor when comparing different molecules in the same sample. Only absolute quantification could illustrate the alterations in cytokines compared to one another as manifest by the protein levels.

Table 4: Actual values obtained for CBA and absolute cytokine analysis

| Cytometric Bead Array values (pg/ml) | | | | |
| --- | --- | --- | --- | --- |
| | IFN$\gamma$ | TNF-$\alpha$ | IL10 | IL2 |
| Medium | 7 | 3 | 8 | 2 |
| ConA | 3082 | 1374 | 895 | 152 |
| PMA+Ionomycin | 17547 | 8198 | 460 | 16088 |
| LPS | 103 | 702 | 4311 | 0 |

| Absolute cDNA levels (Copy No./$\mu$g cDNA) | | | | |
| --- | --- | --- | --- | --- |
| | IFN$\gamma$ | TNF-$\alpha$ | IL10 | IL2 |
| Medium | 6.38E+04 | 8.47E+05 | 4.35E+05 | 9.94E+04 |
| ConA | 6.61E+06 | 8.77E+06 | 2.45E+06 | 1.03E+06 |
| PMA + Ionomycin | 2.60E+08 | 3.63E+06 | 5.43E+05 | 1.15E+08 |
| LPS | 6.75E+04 | 6.57E+05 | 6.81E+06 | 2.65E+04 |

Sensitivity of the absolute quantification protocol

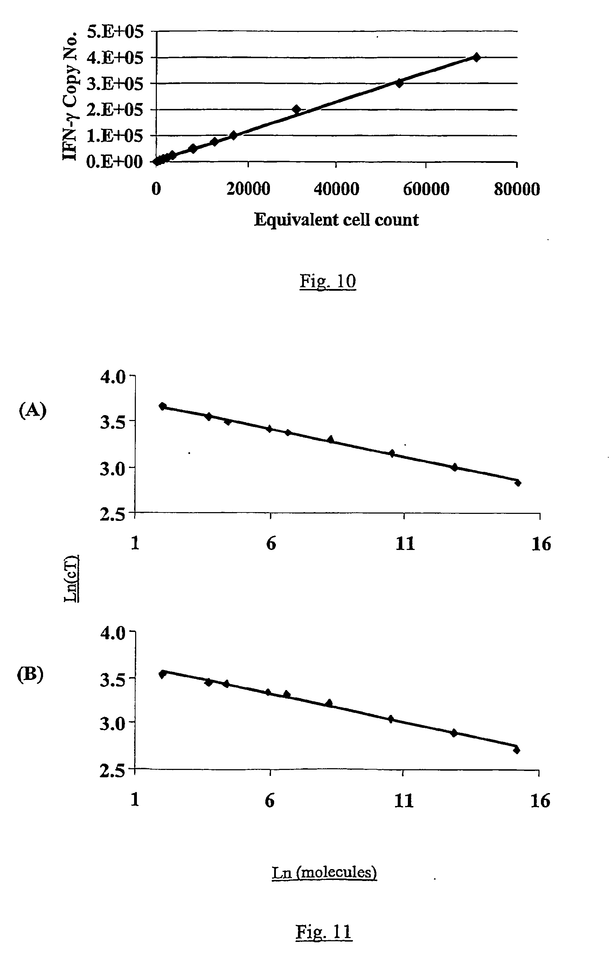

Being a PCR based method, the sensitivity allows minimal cDNA to be used as template and hence screening for a number of cytokines concurrently is feasible. Indeed, it may even be possible to refine this method to multiplex several targets within the same sample, thus examining $T_H1/T_H2$ ratios in the same tube. Fig. 10 shows that IFN-$\gamma$ cDNA may be detected from as few as 1000 cell equivalents. (In Fig. 10, cDNA serially diluted down to a level equivalent of 1000 cells was amplified simultaneously with serially diluted IFN-$\gamma$ plasmid standard in order to calculate copy number. All reactions were done in triplicate. $R^2$ = 0.9966.) Furthermore Fig. 11 demonstrates this method is sensitive down to 8 molecules of the target (i.e. a plasmid dilution of 1 in $5 \times 10^9$). The linear equation remains relatively unchanged between circular and linearised plasmid. Thus Fig. 11 also shows the efficiency of the PCR reaction is not altered by using circular plasmid DNA (Fig. 11A) in place of linearised plasmid DNA (Fig. 11B). Thus, plasmid may be stored in its more stable circular form without the need for cutting with restriction enzymes prior to PCR.

**Discussion**

Real-time PCR is an extremely powerful technique. However, often its results are open to interpretation since there is no convention for data presentation. This anomaly has arisen because many applications rely on non-standard calibration genes, which themselves often change in value during experimental manipulation.
We demonstrate here a preferred method for absolute quantitation of cDNA species using a combination of extremely accurate double-stranded DNA quantitation and a plasmid reference curve. PicoGreen and reference standards are used in the preferred embodiment to measure the amount of cDNA present in a sample using fluorescence. Real-time PCR products are cloned into plasmids and then used to calibrate unknown samples. This cloning is preferably achieved using the same primers necessary for real-time PCR and thus does not involve a second design stage. Thus, results are expressed as copy number/$\mu$g of cDNA and consequently may be compared between both experiments and samples. We present results in Example 1 from a model murine system in which absolute levels of interferon-$\gamma$ and interleukin-4 are measured. In Example 2, we present results from a human system in which absolute levels of interferon-$\gamma$, TNF-$\alpha$, interleukin-2 and interleukin-10 are measured. However, we believe that this technique is widely applicable to the majority of real-time PCR applications.

In the preferred embodiment, the ability to accurately quantitate mRNA species in terms of copy-number represents a major step forward in clinical analysis. It should allow an accurate snapshot of events occurring in vivo to be assessed directly and consequently has multiple applications. The data presented here shows that it is indeed possible to measure absolute numbers of mRNA molecules using real-time PCR in a straightforward manner. By expressing results as copy numbers per $\mu$g of cDNA we have overcome one of the major problems with previous techniques, since it is now possible to compare samples directly, regardless of the efficiency of mRNA synthesis. Our experience in cancer patient clinical trials has shown that T-cell numbers drop markedly with advancing disease. Hence, the ability to express results regardless of mRNA levels is a major step forward. We present data purely from an artificial system, to demonstrate proof of principle, but the technique is widely applicable to a variety of situations.

An important aspect of this method has been to utilise a sensitive and reproducible method for ds-DNA quantitation. Unfortunately, many laboratories do not employ rigorous enough methods to ensure that ds-template is accurately measured and therefore it is unlikely that their results can be used in comparative studies. Although the use of PicoGreen is, in itself, not unique (Singer *et al,* 1997), this is the first time it has been coupled with real-time PCR to calibrate copy-number calculations.

Most of real-time PCR data in the literature is derived from the use of relative quantification by employing calibration genes. One common gene used for calibration is glyceraldehyde phosphate dehydrogenase (GAPDH) (Overbergh *et al,* 1999) which is found in the glycolysis pathway. Unfortunately, although it has become common practice to normalise levels of test genes against this, it has been shown that the expression of GAPDH is regulated by interleukin-2 (Sabath *et al,* 1990). Consequently, in any inflammatory condition, the levels of GAPDH will alter and thus render relative quantitation invalid. This is clearly demonstrated in Fig. 9. Other calibration genes have also been used and one of the most recent is ribosomal RNA (Pleau *et al,* 2001). Whilst this may be superior to others, arguments can still be made to its validity (Schmittgen and Zakrajsek, 2000). Recent data suggests that relative quantitation may be even more unreliable since it has been shown that the reference gene must be at a similar concentration to the unknown in order for results to be valid (Ke *et al,* 2000). Obviously, for clinical applications this is clearly unacceptable since the outcome is completely unknown.

The use of cloned targets is known in real-time applications and allows absolute copy-number to be evaluated by use of a standard curve (Li and Wang, 2000). However, our preferred method has been designed so that it is relatively simple to create a specific plasmid for each mRNA target by using the same primers for real-time PCR as for cloning. After DNA quantitation, the plasmid is available for absolute quantitation of cDNA copy-number. Furthermore, we show that results are the same for both linear and circular plasmids, thus facilitating reference plasmid storage. The simplicity of this technique makes the use of reference genes, and their associated complexity, redundant.

Our particular interest has been to use the technique to measure cytokine transcripts from cancer patient samples before and after vaccination. The measurement of cytokines is a major area of research, and several authors have measured transcripts using real-time PCR (Stordeur *et al,* 2002; Overbergh *et al,* 1999; Kruse *et al,* 1997). Consequently, this topic requires standardisation since several different methods are employed. We propose that by using copy number per $\mu$g cDNA it should be possible to compare results between laboratories. Furthermore, this particular application demonstrates another advantage of the technique, since it is possible to use the same cells for both mRNA and protein quantitation.

Routinely, we stimulate cells overnight with mitogens or Calcium ionophore, harvest cells for mRNA extraction and retain the supernatant. This culture medium can then be tested for protein content by a variety of techniques such as ELISA or Cytometric Bead Array (CBA; BD Biosciences). We believe that this represents a major step forward since, although it it well known mRNA levels do not correlate absolutely with protein (Hein *et al,* 2001), an ability to prove that the changes detected in message levels correlate with the protein output of the cell, gives functional validity to real-time data. Although total levels do vary slightly (Table 4), clear trends in both systems are apparent (Fig. 9). Both real-time PCR and CBA are plate based assays and thus have a high throughput making them ideal for use in clinical trial monitoring.

References

1. Santagati,S., Gamier,M., Carlo,P., Violani,E., Picotti,G.B., and Maggi,A. 1997 Quantitation of low abundance mRNAs in glial cells using different polymerase chain reaction (PCR)-based methods. Brain Res.Brain Res.Protoc. 1, 217-223.

2. Holland,P.M., Abramson,R.D., Watson,R., and Gelfand,D.H. 1991 Detection of specific polymerase chain reaction product by utilizing the 5'----3' exonuclease activity of Thermus aquaticus DNA polymerase. Proc.Natl.Acad.Sci.U.S.A88, 7276-7280.

3. Livak,K.J., Flood,S.J., Marmaro,J., Giusti,W., and Deetz,K. 1995 Oligonucleotides with fluorescent dyes at opposite ends provide a quenched probe system useful for detecting PCR product and nucleic acid hybridization. PCR Methods Appl.4, 357-362.

4. Overbergh,L., Valckx,D., Waer,M., and Mathieu,C. 1999 Quantification of murine cytokine mRNAs using real time quantitative reverse transcriptase PCR. Cytokine11, 305-312.

5. Li,X. and Wang,X. 2000 Application of real-time polymerase chain reaction for the quantitation of interleukin-1beta mRNA upregulation in brain ischemic tolerance. Brain Res.Brain Res.Protoc.5, 211-217.

6. Di Marzio,P., Puddu,P., Conti,L., Belardelli,F., and Gessani,S. 1994 Interferon gamma upregulates its own gene expression in mouse peritoneal macrophages. J Exp.Med.179, 1731-1736.

7. Koch,K.C., Ye,K., Clark,B.D., and Dinarello,C.A. 1992 Interleukin 4 (IL) 4 upregulates gene and surface IL 1 receptor type I in murine T helper type 2 cells. Eur.J Immuno122, 153-157.

8. Singer,V.L., Jones,L.J., Yue,S.T., and Haugland,R.P. 1997 Characterization of PicoGreen reagent and development of a fluorescence- based solution assay for double-stranded DNA quantitation. Anal.Biochem.249, 228-238.

9. Sabath,D.E., Broome,H.E., and Prystowsky,M.B. 1990 Glyceraldehyde-3-phosphate dehydrogenase mRNA is a major interleukin 2- induced transcript in a cloned T-helper lymphocyte. Gene 91, 185-191.

10. Pleau,J.M., Esling,A., Geutkens,S., Dardenne,M., and Homo-Delarche,F. 2001 Pancreatic hormone and glutamic acid decarboxylase expression in the mouse thymus: a real-time PCR study. Biochem.Biophys.Res.Commun.283, 843-848.

11. Schmittgen,T.D. and Zakrajsek,B.A. 2000 Effect of experimental treatment on housekeeping gene expression: validation by real-time, quantitative RT-PCR. J Biochem.Biophys.Methods 46, 69-81.

12. Ke,L.D., Chen,Z., and Yung,W.K. 2000 A reliability test of standard-based quantitative PCR: exogenous vs endogenous standards. Mol.Cell Probes14, 127-135.

13. Stordeur,P., Poulin,L.F., Craciun,L., Zhou,L., Schandene,L., de Lavareille,A., Goriely,S., and Goldman,M. 2002 Cytokine mRNA quantification by real-time PCR. J Immunol Methods 259, 55-64.

14. Kruse,N., Pette,M., Toyka,K., and Rieckmann,P. 1997 Quantification of cytokine mRNA expression by RT PCR in samples of previously frozen blood. J Immunol Methods 210, 195-203.

15. Hein,J., Schellenberg,U., Bein,G., and Hackstein,H. 2001 Quantification of murine IFN-gamma mRNA and protein expression: impact of real-time kinetic RT-PCR using SYBR green I dye. Scand.J Immunol 54, 285-291.

## Claims

1.  A method for quantification of a target nucleic acid in a sample, comprising the steps of:

    (1) accurately quantifying the total amount of nucleic acid in the sample using PicoGreen® dsDNA quantitation reagent in combination with a known reference standard;
    (2) amplifying the target nucleic acid using real-time PCR primers and probes to produce an amount of amplicon;
    (3) building a reference plasmid based on this amplicon;
    (4) constructing a fluorescence reference curve of real-time PCR reactions with different known quantities of the plasmid using a fluorescent probe;
    (5) running a real-time PCR reaction with an aliquot of the sample to amplify the target nucleic acid and obtaining a fluorescence result from the binding of a fluorescent probe; and
    (6) correlating the fluorescence result of step (5) with the reference curve of step (4) to quantify the target nucleic acid by copy number per amount of total nucleic acid present in the sample (for example, copy number per $\mu$g cDNA).

2.  The method according to claim 1, wherein the amplicon of step (2) is cloned into a vector in step (3) to produce the plasmid which is quantified and used to construct the fluorescence reference curve of step (4).

3.  The method according to either one of claim 1 or claim 2, wherein the real-time PCR reaction in step (5) employs the real-time primers used in step (2).

4.  The method according to any of claims 1 to 3, wherein both step (2) and step (5) are carried out using a fluorescence-detecting thermocycler, for example a Perkin Elmer/Applied Biosystems Prism 7700 Sequence Detector System®.

5.  The method according to any one of the preceding claims, wherein the fluorescence reference curve is constructed using the natural log of the threshold cycle ($C_T$) of the real-time PCR reactions against given quantities of the amplicon or cloned amplicon expressed as copy number.

6.  The method according to any one of the preceding claims, wherein the target nucleic acid comprises RNA.

7.  The method according to any one of the preceding claims, wherein cDNA constructed from the RNA is used as the target nucleic acid in steps (2) and (5).

8.  The method according to any one of the preceding claims, wherein the target nucleic acid used in step (2) is from a source other than the sample.

9. The method according to any one of the preceding claims, wherein steps (4) and (5) are conducted in same reaction plate.

10. The method according to any one of the preceding claims, wherein the quantity of target nucleic acid is proportional to a cytokine quantity.

11. The method according to claim 10, wherein the real-time PCR reactions employ pre-defined assay reagents (PDAR) specific for IFN-γ or IL4 (for example, as obtainable from Applied Biosystems).

12. The method according to claim 10, wherein the real-time PCR reactions use primers and probes for IFN-γ (Primers: SEQ ID Nos. 1 & 2, Probe: SEQ ID NO. 3), IL2 (Primers: SEQ ID Nos. 4 & 5, Probe: SEQ ID NO. 6), IL10 (Primers: SEQ ID Nos. 7 & 8, Probe: SEQ ID NO. 9) and/or TNF-α (Primers: SEQ ID Nos. 10 & 11, Probe: SEQ ID NO. 12).

**Patentansprüche**

1. Verfahren zur Quantifizierung einer Zielnucleinsäure in einer Probe, das die folgenden Schritte beinhaltet:

(1) genaues Quantifizieren der Gesamtmenge von Nucleinsäure in der Probe unter Verwendung des PicoGreen® dsDNA Mengenbestimmungsreagens in Kombination mit einem bekannten Referenzstandard;
(2) Amplifizieren der Zielnucleinsäure unter Verwendung von Echtzeit-PCR-Primern und -Sonden zum Produzieren einer Amplicon-Menge;
(3) Errichten eines Referenzplasmids auf der Basis dieses Amplicons;
(4) Konstruieren einer Fluoreszenzreferenzkurve von Echtzeit-PCR-Reaktionen mit verschiedenen bekannten Quantitäten des Plasmids unter Verwendung einer Fluoreszenzsonde;
(5) Durchführen einer Echtzeit-PCR-Reaktion mit einem Aliquot der Probe, um die Zielnucleinsäure zu amplifizieren, und Erhalten eines Fluoreszenzergebnisses von der Bindung einer Fluoreszenzsonde; und
(6) Korrelieren des Fluoreszenzergebnisses aus Schritt (5) mit der Referenzkurve aus Schritt (4), um die Zielnucleinsäure in Kopiezahl je Menge an in der Probe vorliegender Gesamtnucleinsäure zu quantifizieren (zum Beispiel Kopiezahl je μg cDNA).

2. Verfahren nach Anspruch 1, wobei das Amplicon aus Schritt (2) in einen Vektor im Schritt (3) kloniert wird, um das Plasmid zu produzieren, das quantifiziert und zum Konstruieren der Fluoreszenzreferenzkurve aus Schritt (4) verwendet wird.

3. Verfahren nach Anspruch 1 oder Anspruch 2, wobei in der Echtzeit-PCR-Reaktion aus Schritt (5) die im Schritt (2) verwendeten Echtzeit-Primer eingesetzt werden.

4. Verfahren nach einem der Ansprüche 1 bis 3, wobei sowohl Schritt (2) als auch Schritt (5) mit einem Fluoreszenz nachweisenden Thermocycler, z.B. Prism 7700 Sequence Detector System® von Perkin Elmer/Applied Biosystems, durchgeführt wird.

5. Verfahren nach einem der vorherigen Ansprüche, wobei die Fluoreszenzreferenzkurve unter Verwendung des natürlichen Logarithmus des Schwellenzyklus ($C_T$) der Echtzeit-PCR-Reaktionen gegenüber gegebenen Quantitäten des Amplicons oder klonierten Amplicons, ausgedrückt als Kopiezahl, konstruiert wird.

6. Verfahren nach einem der vorherigen Ansprüche, wobei die Zielnucleinsäure RNA beinhaltet.

7. Verfahren nach einem der vorherigen Ansprüche, wobei von der RNA konstruierte cDNA als die Zielnucleinsäure in den Schritten (2) und (5) verwendet wird.

8. Verfahren nach einem der vorherigen Ansprüche, wobei die in Schritt (2) verwendete Zielnucleinsäure von einer anderen Quelle als der Probe stammt.

9. Verfahren nach einem der vorherigen Ansprüche, wobei die Schritte (4) und (5) in derselben Reaktionsplatte durchgeführt werden.

**10.** Verfahren nach einem der vorherigen Ansprüche, wobei die Quantität der Zielnucleinsäure proportional zur Cytokin-Quantität ist.

**11.** Verfahren nach Anspruch 10, wobei die Echtzeit-PCR-Reaktionen für IFN-γ oder IL4 spezifische vordefinierte Assay-Reagenzien (PDAR) einsetzen (wie z.B. von Applied Biosystems erhältlich).

**12.** Verfahren nach Anspruch 10, wobei für die Echtzeit-PCR-Reaktionen Primer und Sonden für IFN-γ (Primer: SEQ ID Nr. 1 & 2, Sonde: SEQ ID Nr. 3), IL2 (Primer: SEQ ID Nr. 4 & 5, Sonde: SEQ ID Nr. 6), IL10 (Primer: SEQ ID Nr. 7 & 8, Sonde: SEQ ID Nr. 9) und/oder TNF-α (Primer: SEQ ID Nr. 10 & 11, Sonde: SEQ ID NR. 12) verwendet werden.

**Revendications**

**1.** Procédé de quantification d'un acide nucléique cible dans un échantillon, comprenant les étapes consistant à :

(1) quantifier précisément la quantité totale d'acide nucléique dans l'échantillon en utilisant le réactif de quantification d'ADN-db PicoGreen® en association avec un étalon de référence connu;
(2) amplifier l'acide nucléique cible en utilisant des amorces et des sondes de PCR en temps réel pour produire une quantité d'amplicon;
(3) synthétiser un plasmide de référence basé sur cet amplicon;
(4) établir une courbe de référence de fluorescence de réactions de PCR en temps réel avec différentes quantités connues du plasmide en utilisant une sonde fluorescente;
(5) effectuer une réaction de PCR en temps réel avec une aliquote de l'échantillon pour amplifier l'acide nucléique cible et obtenir un résultat de fluorescence de la liaison d'une sonde fluorescente; et
(6) mettre en corrélation le résultat de fluorescence de l'étape (5) avec la courbe de référence de l'étape (4) pour quantifier l'acide nucléique cible par nombre de copies par quantité d'acide nucléique total présent dans l'échantillon (par exemple, nombre de copies par μg d'ADNc).

**2.** Le procédé selon la revendication 1, dans lequel l'amplicon de l'étape (2) est cloné dans un vecteur à l'étape (3) pour produire le plasmide qui est quantifié et utilisé pour établir la courbe de référence de fluorescence de l'étape (4).

**3.** Le procédé selon la revendication 1 ou la revendication 2, dans lequel la réaction de PCR en temps réel de l'étape (5) utilise les amorces en temps réel de l'étape (2).

**4.** Le procédé selon l'une quelconque des revendications 1 à 3, dans lequel l'étape (2) et l'étape (5) sont effectuées toutes les deux en utilisant un thermocycleur de détection de fluorescence, par exemple un Perkin Elmer/Applied Biosystems Prism 7700 Sequence Detector System®.

**5.** Le procédé selon l'une quelconque des revendications précédentes, dans lequel la courbe de référence de fluorescence est établie en utilisant le logarithme naturel du cycle seuil ($C_T$) des réactions de PCR en temps réel, contre des quantités données de l'amplicon ou de l'amplicon cloné, exprimées en tant que nombre de copies.

**6.** Le procédé selon l'une quelconque des revendications précédentes, dans lequel l'acide nucléique cible comprend de l'ARN.

**7.** Le procédé selon l'une quelconque des revendications précédentes, dans lequel l'ADNc synthétisé de l'ARN, est utilisé comme l'acide nucléique cible aux étapes (2) et (5).

**8.** Le procédé selon l'une quelconque des revendications précédentes, dans lequel l'acide nucléique cible utilisé à l'étape (2) provient d'une source autre que l'échantillon.

**9.** Le procédé selon l'une quelconque des revendications précédentes, dans lequel les étapes (4) et (5) sont effectuées sur la même plaque de réaction.

**10.** Le procédé selon l'une quelconque des revendications précédentes, dans lequel la quantité d'acide nucléique cible est proportionnelle à une quantité de cytokine.

**11.** Le procédé selon la revendication 10, dans lequel les réactions de PCR en temps réel utilisent des réactifs d'essai pré-définis (PDAR), spécifiques pour l'IFN-γ ou l'IL4 (par exemple tels qu'ils peuvent être obtenus auprès de Applied Biosystems).

12. Le procédé selon la revendication 10, dans lequel les réactions de PCR en temps réel utilisent des amorces et des sondes pour IFN-γ (Amorces: SEQ ID NO: 1 & 2, Sonde: SEQ ID NO:3), pour IL2 (Amorces: SEQ ID NO: 4 & 5, Sonde: SEQ ID NO:6), pour IL10 (Amorces: SEQ ID NO: 7 & 8, Sonde: SEQ ID NO: 9) et/ou pour TNF-α (Amorces: SEQ ID NO: 10 & 11, Sonde: SEQ ID NO:12).

Fig. 1

Fig. 2

Fig. 3

Fig. 4

Fig. 5

Fig. 6

Fig. 7

Fig. 8

Fig. 9

Fig. 10

Ln (molecules)

Fig. 11